# EUROPEAN PATENT SPECIFICATION

(11) **EP 2 430 032 B1**
(45) Date of publication and mention of the grant of the patent: **17.02.2016**
(21) Application number: 10721447.0
(22) Date of filing: 12.05.2010
(51) Int. Cl.: C07H 1/00, C07H 19/19

(54) **METHOD FOR THE MANUFACTURE OF 2-FLUORO-ARA-ADENINE**
VERFAHREN ZUR HERSTELLUNG VON 2-FLUOR-ARA-ADENIN
PROCÉDÉ DE FABRICATION DE 2-FLUORO-ARA-ADÉNINE

(30) Priority: 15.05.2009 EP 09160408; 29.09.2009 US 246664 P
(43) Date of publication of application: 21.03.2012
(73) Proprietor: Alcafleu Management GmbH & Co. KG, 15732 Schönefeld OT Waltersdorf (DE)
(72) Inventor: BERWE, Mathias, 45549 Sprockhövel (DE); BOTHE, Clemens, 51375 Leverkusen (DE); REHSE, Joachim, 42799 Leichlingen (DE)
(74) Representative: Savic, Bojan
(86) International application number: PCT/EP2010/056535
(87) International publication number: WO 2010/130778

(56) References cited:
- EP-A1- 1 464 708
- EP-B1- 0 670 845

## Description

The invention relates to a method for the manufacture of pure 2-fluoro-ara-adenine having a reduced contents of dimers from 2-fluoro-ara-adenine triacetate using potassium carbonate (K₂CO₃).

2-Fluoro-ara-adenine is an important precursor in the manufacture of the pharmaceutical active ingredient fludarabine phosphate (Fludara^{®}). Fludarabine phosphate (Fludara^{®}) finds use particularly in the treatment of chronic lymphocytic leukemia (CLL).

In general, the manufacture of fludarabine phosphate (Fludara^{®}) occurs via the following steps: guanosine → 2-amino-adenosine → 2-F-adenosine triacetate, raw → 2-F-adenosine triacetate, purified → 2-F-adenosine diacetate, raw → 2-F-adenosine diacetate, purified → 2-F-adenine triacetate, purified → 2-fluoro-ara-adenine → fludarabine phosphate (Fludara^{®}).

In general, a subsequent purification occurs via the Na salt.

Various method variants have been described for the conversion.

In US 4,188,378, US 4,210,745 and US 4,357,324 as well as in J. Med, Chem. 12, 498 - 504 (1969) and J. Het. Chem 16, 157 - 160 (1979), for example, various methods are described for the manufacture of 2-substituted 9-(2,3,5-tri-O-benzyl-ß-D-arabinofuranosyl) adenine derivatives. Moreover, in US 5,296,589 and US 5,110,919, the manufacture of fludarabine from 2-fluoro-9-(2,3,5-tri-benzyl-β-D-arabinofuranosyl) adenine is described.

In EP 0 578 208, the advantageous manufacture of 2-fluoro-9-(2,3,5-tri-O-benzyl-ß-arabinofuranosyl) adenine is described.

In EP 0 614 462, the manufacture of 2-fluoroadenosine-2',3',5'-tri-O-acetate is described, among other things, which is an intermediate product in the manufacture of fludarabine phosphate (Fludara^{®}).

In EP 0 670 845, the manufacture of fludarabine phosphate (Fludara^{®}) from guanosine is described, wherein the manufacture occurs via 2-fluoro-2',3',5'-tri-O-acyladenosine, among other things.

Tian et al., Huaxue Yanjiu Yingyong Journal, 18 (5), 570-572, 2006, describe a 5-stage method for the manufacture of fludarabine phosphate (Fludara^{®}) using 2,6-bis(acetamido) purine and 2,3,5-tri-O-benzyl-D-arabinofuranosyl chloride.

Xu et al., Jingxi Yu Zhuanyong Huaxuepin (2006), 14(7), 16-18, describe the manufacture of fludarabine phosphate (Fludara^{®}) from 2,6-diaminopurine via 2,6-di(acetamido)-9-(2,3,5-tri-O-benzyl-β-D-arabinofuranosyl)purine.

Li et al., Jiangsu Huagong (2005), 33(Suppl.), 121-123, describe the manufacture of fludarabine from 2-amino-6-chloropurine and 2,3,5-tri-O-benzyl-1-O-p-nitrobenzoyl-beta-D-arabinose.

In EP 0 759 926, the manufacture of fludarabine through enzymatic hydrolysis of the corresponding triacetate with pig liver esterase is described.

In addition to these known reactions, in particular the saponification reactions of 2-fluoro-ara-adenine triacetates to 2-fluoro-ara-adenine, other methods have been described as well such as, for example, the manufacture of fludarabine phosphate (Fludara^{®}) through conversion of 2-fluoro-ara-adenine with phosphoroxychloride (Wang et al., University of Science and Technology, Shanghai, 200237, People's Republic of China, Huadong Ligong Daxue Xuebao, Ziran Kexueban (2006), 32(12), 1436-1439).

An important process stage in the procedure described above is the conversion to 2-fluoro-ara-adenine.

A disadvantage of the known methods is the fact that during the process stage of the conversion to 2-fluoro-ara-adenine, the amount of dimers is decisively determined. Such dimers have the following structures:

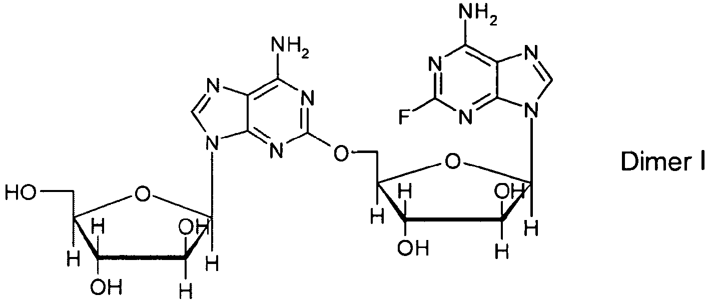

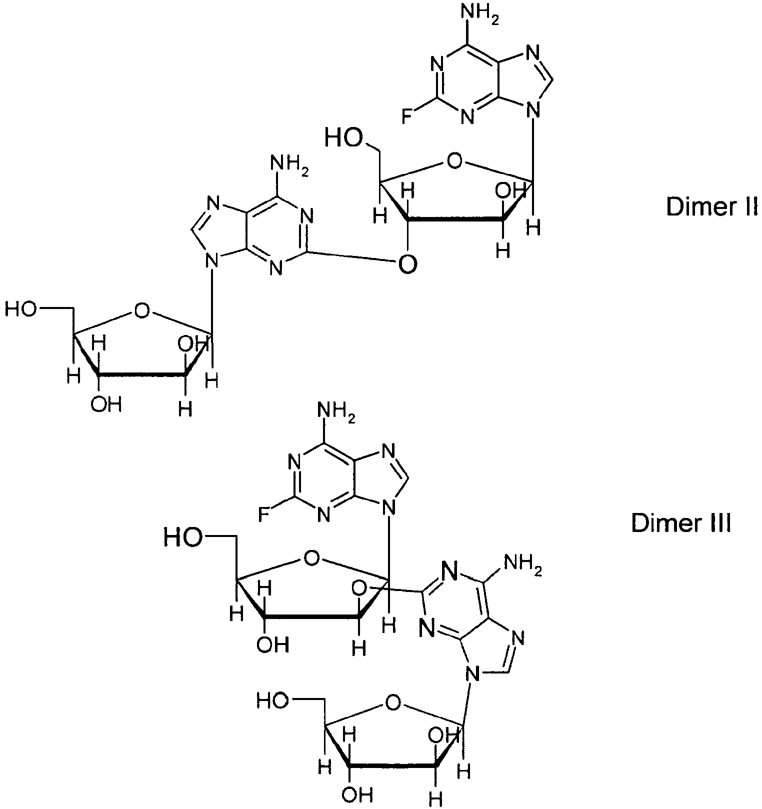

In the subsequent stages of the conversion to fludarabine phosphate (Fludara^{®}), the dimers are often only marginally degraded.

In the conventional method for the manufacture of fludarabine phosphate, saponification of the three acetate groups of the 2-fluoro-ara-adenine triacetate is carried out at 95°C with 4 eq. of sodium hydrogencarbonate (NaHCO₃) in water.

During this process, heavy foam formation occurs in the reactor which is very disadvantageous as well.

The raw product precipitated by means of cooling is isolated and dissolved in ethanol/water under reflux and activated carbon is added. Following hot filtration, 2-fluoro-ara-adenine is crystallized out by cooling and isolated. The dimer contents obtained through this method is relatively high and lies within a range of between 1.5 and 2%. The yield lies at only 77% of the theory.

Another advantage would be the avoidance of an interim isolation of a 2-fluoro-ara-adenine raw product.

EP 0 670 845 B1 relates to a specific process for the preparation of fludarabine phosphate from guanosine. EP 1 464 708 A1 relates to a specific process for the preparation of fludarabine phosphate from 2-fluoroadenine.

Thus, the task of the present invention is to provide a method that overcomes the disadvantages of the known methods, namely
a) limiting the dimer contents to a minimum,
b) avoiding an intermediate isolation of the 2-fluoro-ara-adenine raw product,
c) preventing heavy foam formation in the reactor, and
d) obtaining a higher yield.

It has now been found out that the known disadvantages can be overcome by means of a method for the manufacture of 2-fluoro-ara-adenine of Formula I 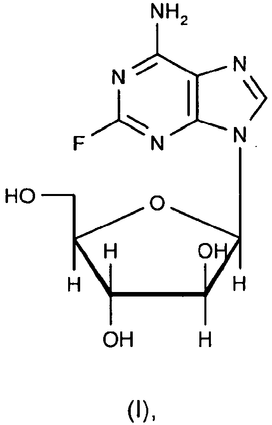 that is characterized in that
a) initially, 2-fluoro-ara-adenine triacetate of Formula II 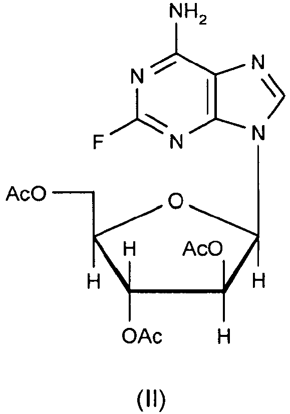 and potassium carbonate (K₂CO₃) in an aqueous alcoholic solution are provided and stirred under heating,
b) the reaction mixture is subsequently cooled down, water is added, and the reaction mixture is adjusted to a suitable pH value,
c) activated carbon is first added to the reaction mixture treated in this way ,and the reaction mixture is kept under reflux at a suitable temperature and subsequently the activated carbon is filtered off the reaction mixture,
d) the filtrate is subsequently inoculated with 2-fluoro-ara-adenine without intermediate isolation of the raw 2-fluoro-ara-adenine, the resulting suspension is cooled down again and stirred further, and
e) finally, the isolated solid cake is washed with an alcoholic aqueous solution and dried at a higher temperature under vacuum using a carrier gas.

In process stages a) and e), any alcohol such as, for example, methanol, ethanol, n-propanol, n-butanol, isopropanol, isobutanol, 2-butanol and tert-butanol may be used for the aqueous alcoholic solution.

Depending on the choice of alcohol, it may be advisable to adjust the reaction temperature and/or the reaction time.

When using methanol, the conversion may be carried out at a temperature of as low as 0°C and with a short reaction time.

When using linear higher alcohols such as, for example, n-propanol or n-butanol, a reaction temperature of 50°C to 60°C may be advisable.

When using branched alcohols such as, for example, isopropanol, 2-butanol or tert-butanol, a reaction temperature of 60°C to 80°C may be advisable.

It is also possible to use the same alcohol during process stages a) and e) or to use an alcohol in process stage a) that is different from that in process stage e).

Preferably, a mixture of linear alcohol and water serves as aqueous alcoholic solution in process stages a) and e).

Particularly preferably, a mixture of ethanol and water serves as aqueous alcoholic solution in process stages a) and e).

The aqueous alcoholic solution is a mixture of 180 to 220 kg of alcohol and 8 to 12 kg of water, preferably a mixture of 195 to 205 kg of alcohol and 9.8 to 10.2 kg of water.

The temperature range utilized in process stage a) may be from 35 to 45°C, preferably from 37 to 43°C.

The amount of potassium carbonate (K₂CO₃) used for conversion in process stage a) amounts to 2.25 to 2.75 kg, preferably 2.45 to 2.55 kg.

The pH value suitable in process stage b) lies from 5 to 9, preferably from 6 to 8.

The adjustment of the pH value in process stage b) can be carried out with any organic or mineral acid.

Acids suitable for this purpose are in particular, for example, formic acid, acetic acid, sulfuric acid, hydrochloric acid and nitric acid.

Preferably, the pH value is adjusted with acetic acid.

The temperature range suitable for the inoculation in process stage d) should lie at 40 to 75 °C, preferably at 50 to 60 °C and particularly preferably at 52 to 58 °C.

The inoculation with 2-fluoro-ara-adenine carried out in process stage d) can be done in an amount of 0.05 to 1 kg, preferably in an amount of 0.05 to 0.2 kg.

For the drying process, any gas may be used as carrier gas; preferably, nitrogen will be used.

The advantages of the new method vis-a-vis the methods known from the state of the art are a lower dimer contents due to the milder saponification conditions, an omission of the intermediate isolation of the raw product, the avoidance of foam formation as well as an increase in the yield.

The method in accordance with the invention can run on a lab scale as well as preferably under industrial conditions. Under industrial conditions, 2-fluoro-ara-adenine can be produced in an amount of at least 1 kg.

While with the known methods the dimer contents lies within a range of about 1.5 to 2%, the dimer contents can be considerably reduced with the method in accordance with the invention and lies at a total of ≤ 0,3 %.

The disclosure thus also relates to a 2-fluoro-ara-adenine with a dimer contents of ≤ 0,3 %.

In particular, the disclosure relates to 2-fluoro-ara-adenine with a dimer contents of ≤ 0,3 % relative to dimers I to III mentioned above.

The disclosure thus also relates to a 2-fluoro-ara-adenine with a degree of purity of at least 99.7% relative to the portion of dimers, in particular to dimers I to III.

The 2-fluoro-ara-adenine of Formula I produced in this manner, with a dimer portion of ≤ 0,3 %, preferably serves as a valuable intermediate product for the manufacture of fludarabine phosphate, in particular for the manufacture of fludarabine phosphate with a degree of purity of at least 99.5%.

In the following, the manufacture of 2-fluoro-ara-adenine with varying amounts and under different physical conditions will be described, and a comparison will be made showing the advantages of the method in accordance with the invention vis-a-vis the known methods, with the manufacture not being limited to these examples alone.

### Example 1

### Manufacture of 2-fluoro-ara-adenine

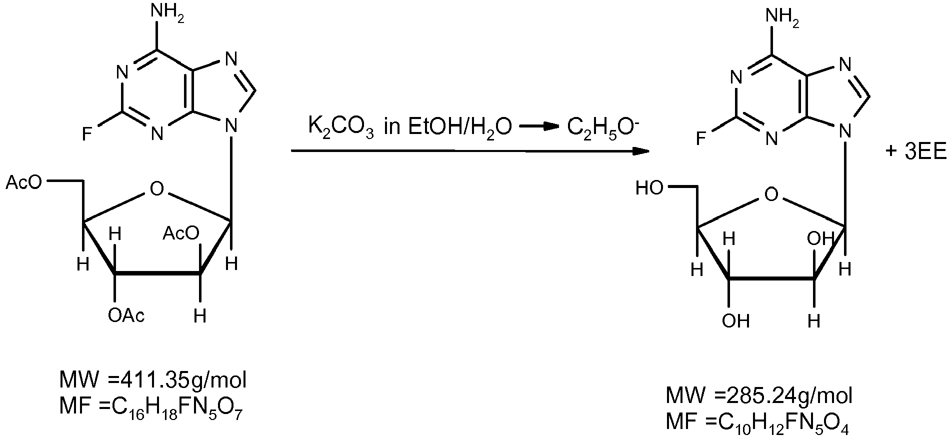

180 to 220 kg of ethanol are provided in an agitator container and 8 to 12 kg of purified water are added. 15 kg of 2-fluoro-ara-adenine triacetate and 2.25 to 2.75 kg potassium carbonate are added. This mixture is heated to 35 to 45 °C and stirred for 20 to 48 h. The reaction mixture is cooled down to 0 to 20 °C and 190 to 230 kg of purified water are added. Subsequently, the pH value is adjusted to a value of 5 to 7 using an organic or mineral acid, preferably acetic acid. Activated carbon is added to the reaction mixture and the reaction mixture is brought to reflux. The activated carbon is filtered off while warm and

the filtrate is inoculated with 2-fluoro-ara-adenine within a temperature range of 40 to 75 °C. The inoculated amount lies within a range of 0 to 1 kg. The resulting suspension is cooled down to -12 to 10 °C and stirred for at least an additional 60 min. The isolated solid cake is washed with a mixture of ethanol and purified water and dried under vacuum at a preferred shell temperature of 80 to 100 °C using a carrier gas.

The yield lies at 82% of the theory.

### Example 2

### Manufacture of 2-fluoro-ara-adenine

195 to 205 kg of ethanol are provided in an agitator container and 9.8 to 10.2 kg of purified water are added. 15 kg of 2-fluoro-ara-adenine triacetate and 2.45 to 2.55 kg of potassium carbonate are added. The mixture is heated to 37 to 43 °C and stirred for 23-25 h. The reaction mixture is cooled down to 0 to 10 °C and 195 to 205 kg of purified water are added. Subsequently, the pH value is adjusted to a value of 6 to 7 using an organic or mineral acid, preferably acetic acid. Activated carbon is added to the reaction mixture, and the reaction mixture is brought to reflux. The activated carbon is filtered off while warm and the filtrate is inoculated with 2-fluoro-ara-adenine within a temperature range of 50 to 60 °C. The inoculated amount lies within a range of 0 to 0.2 kg. The resulting suspension is cooled down to -8 to -2 °C and stirred for at least an additional 60 min. The isolated solid cake is washed with a mixture of ethanol and purified water and dried under vacuum at a shell temperature of 80 to 100 °C using a carrier gas.

The yield lies at 82% of the theory.

### Example 3

### Manufacture of 2-fluoro-ara-adenine

195 to 205 kg of ethanol are provided in an agitator container and 9.8 to 10.2 kg of purified water are added. 15 kg of 2-fluoro-ara-adenine triacetate and 2.45 to 2.55 kg of potassium carbonate are added. The mixture is heated to 37 to 43 °C and stirred for 23-25 h. The reaction mixture is cooled down to 0 to 10 °C and 195 to 205 kg of purified water are added. Subsequently, the pH value is adjusted to a value of 6 to 7 using an organic or mineral acid, preferably acetic acid. Activated carbon is added to the reaction mixture, and the reaction mixture is brought to reflux. The activated carbon is filtered off while warm and the filtrate is inoculated with 2-fluoro-ara-adenine within a temperature range of 52 to 58 °C. The inoculated amount lies within a range of 0 to 0.2 kg. The resulting suspension is cooled down to -8 to -2 °C and stirred for at least an additional 60 min. The isolated solid cake is washed with a mixture of ethanol and purified water and dried under vacuum at a shell temperature of 80 to 100 °C using a carrier gas.

The yield lies at 82% of the theory.

### Example 4

### Comparison of the conventional preparation method with the method in accordance with the invention

| | **% Dimers** | **% Yield** |
|---|---|---|
| Conventional method with saponification at 95°C with 4 eq. NaHCO₃ in water | 1.6 | 77 |
| Method in accordance with the invention | 0.3 | 82 |

The result of the comparison clearly shows the superiority of the method in accordance with the invention vis-a-vis the known methods.

## Claims

1. A method for the manufacture of 2-fluoro-ara-adenine of Formula I 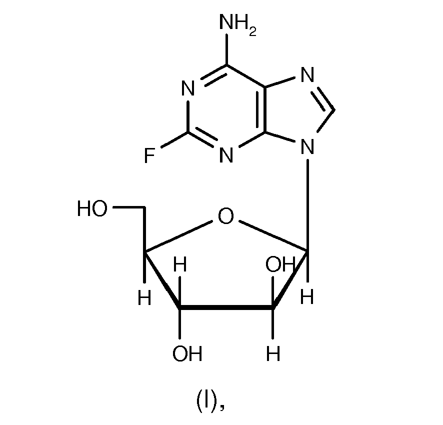 **characterized in that**
a) initially 2-fluoro-ara-adenine triacetate of Formula II 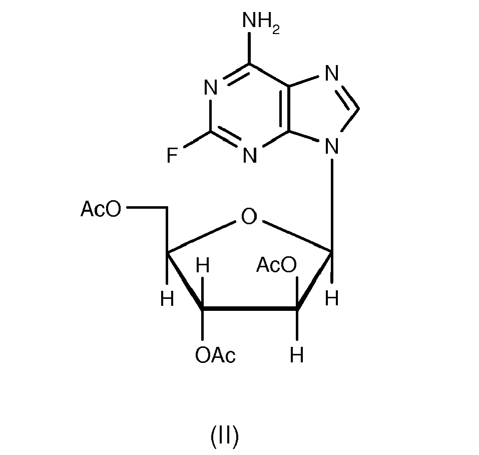 and 2.25 to 2.75 kg or 2.45 to 2.55 kg of potassium carbonate (K₂CO₃) in an aqueous alcoholic solution are provided and stirred under heating, wherein the said aqueous alcoholic solution is a mixture of 180 to 220 kg of alcohol and 8 to 12 kg of water or a mixture of 195 to 205 kg of alcohol and 9.8 to 10.2 kg of water,
b) the reaction mixture is subsequently cooled down, water is added, and the reaction mixture is adjusted to a pH value of 5 to 9 or 6 to 8,
c) activated carbon is first added to the reaction mixture treated in this manner, and the reaction mixture is kept under reflux and subsequently the activated carbon is filtered off the reaction mixture,
d) the filtrate is subsequently inoculated with 2-fluoro-ara-adenine without intermediate isolation of the raw 2-fluoro-ara-adenine, the resulting suspension is cooled down again and stirred further, and
e) finally, the isolated solid cake is washed with an alcoholic aqueous solution and dried at a higher temperature under vacuum using a carrier gas.

2. The method in accordance with Claim 1, **characterized in that** in method stages a) and e) a mixture of ethanol and water are used as the aqueous alcoholic solution.

3. The method in accordance with one of Claims 1 and 2,
**characterized in that** in process stage a) work is done within a temperature range of 35 to 45°C; or
**characterized in that** in process stage a) work is done within a temperature range of 37 to 43°C.

4. The method in accordance with one of Claims 1 through 3,
**characterized in that** in process stage d) the inoculation is carried out within a temperature range of 40 to 75 °C; or
**characterized in that** in process stage d) the inoculation is carried out within a temperature range of 50 to 60 °C; or
**characterized in that** in method stage d) the inoculation is carried out within a temperature range of 52 to 58 °C.

5. The method in accordance with one of Claims 1 through 4,
**characterized in that** in process stage d) the inoculation with 2-fluoro-ara-adenine is carried out in an amount of 0.05 to 1 kg; or
**characterized in that** in process stage d) the inoculation with 2-fluoro-ara-adenine is carried out in an amount of 0.05 to 0.2 kg.

## Patentansprüche

1. Verfahren zur Herstellung eines 2-Fluor-ara-adenins nach Formel I 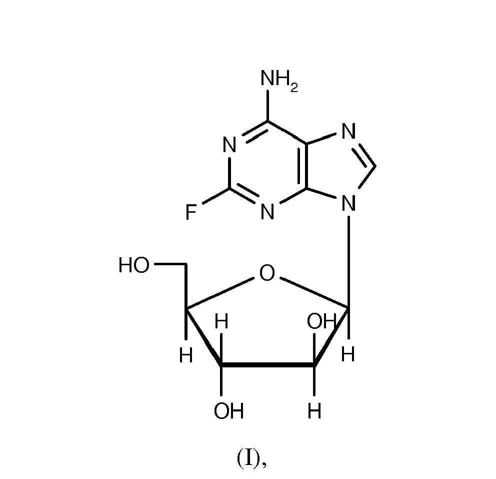 **dadurch gekennzeichnet, dass**
a) zuerst 2-Fluor-ara-adenintriacetat nach Formel II 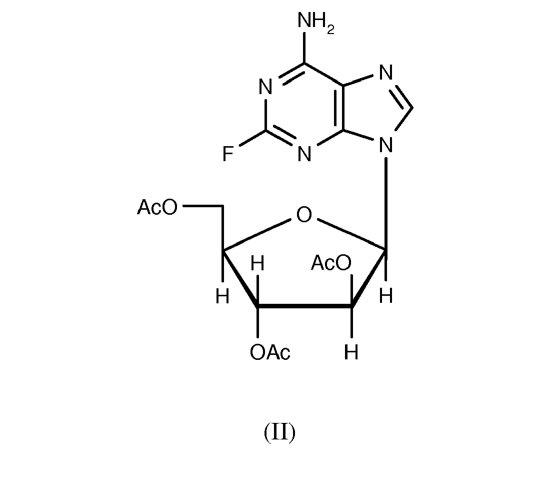 und 2.25 bis 2.75 kg oder 2.45 bis 2.55 kg Kaliumcarbonat (K₂CO₃) in einer wässrigen alkoholischen Lösung bereitgestellt und unter Erwärmen gerührt werden, wobei die wässrige alkoholische Lösung eine Mischung von 180 bis 220 kg Alkohol und 8 bis 12 kg Wasser oder eine Mischung von 195 bis 205 kg Alkohol und 9.8 bis 10.2 kg Wasser ist,
b) die Reaktionsmischung anschließend abgekühlt wird, Wasser hinzugegeben wird und die Reaktionsmischung auf einen pH Wert von 5 bis 9 oder 6 bis 8 angepasst wird,
c) Aktivkohle zunächst zu der so behandelten Reaktionsmischung hinzugegeben wird und die Reaktionsmischung unter Rückfluss gehalten wird und anschließend die Aktivkohle aus der Reaktionsmischung abfiltriert wird,
d) das Filtrat anschließend mit 2-Fluor-ara-adenin ohne zwischenzeitliche Isolierung des rohen 2-Fluor-ara-adenins geimpft wird, wobei die resultierende Suspension erneut abgekühlt und weiter gerührt wird, und
e) schließlich der isolierte feste Kuchen mit einer wässrigen alkoholischen Lösung gewaschen wird und unter Verwendung eines Trägergases bei einer höheren Temperatur unter Vakuum getrocknet wird.

2. Verfahren gemäß Anspruch 1, **dadurch gekennzeichnet, dass** bei den Verfahrensstufen a) und e) eine Mischung von Ethanol und Wasser als die wässrige alkoholische Lösung verwendet wird.

3. Verfahren gemäß einem der Ansprüche 1 und 2,
**dadurch gekennzeichnet, dass** bei der Verfahrensstufe a) die Umsetzung in einem Temperaturbereich von 35 bis 45 °C durchgeführt wird; oder
**dadurch gekennzeichnet, dass** bei der Verfahrensstufe a) die Umsetzung in einem Temperaturbereich von 37 bis 43 °C durchgeführt wird.

4. Verfahren gemäß einem der Ansprüche 1 bis 3,
**dadurch gekennzeichnet, dass** bei der Verfahrensstufe d) das Impfen in einem Temperaturbereich von 40 bis 75 °C durchgeführt wird; oder
**dadurch gekennzeichnet, dass** bei der Verfahrensstufe d) das Impfen in einem Temperaturbereich von 50 bis 60 °C durchgeführt wird; oder
**dadurch gekennzeichnet, dass** bei der Verfahrensstufe d) das Impfen in einem Temperaturbereich von 52 bis 58 °C durchgeführt wird.

5. Verfahren gemäß einem der Ansprüche 1 bis 4,
**dadurch gekennzeichnet, dass** bei der Verfahrensstufe d) das Impfen mit 2-Fluor-ara-adenin in einer Menge von 0.05 bis 1 kg durchgeführt wird; oder
**dadurch gekennzeichnet, dass** bei der Verfahrensstufe d) das Impfen mit 2-Fluor-ara-adenin in einer Menge von 0.05 bis 0.2 kg durchgeführt wird.

## Revendications

1. Procédé pour la préparation de la 2-fluoro-ara-adénine répondant à la formule I : 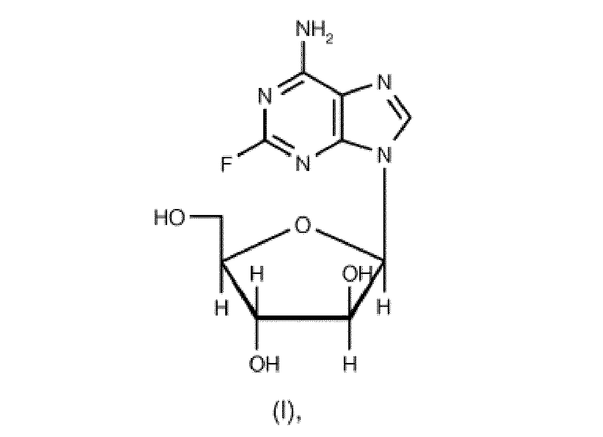 **caractérisé en ce que**
a) dans un premier temps, on procure du triacétate de 2-fluoro-ara-adénine répondant à la formule II 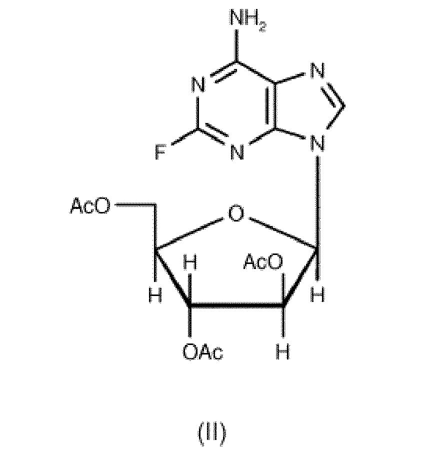 et de 2,25 à 2,75 kg ou de 2,45 à 2,55 kg de carbonate de potassium (K₂CO₃) dans une solution alcoolique aqueuse et on agite tout en chauffant, ladite solution alcoolique aqueuse représentant un mélange de 180 à 220 kg d'alcool et de 8 à 12 kg d'eau ou un mélange de 195 à 205 kg d'alcool et de 9,8 à 10,2 kg d'eau ;
b) on refroidit ensuite le mélange réactionnel, on ajoute de l'eau et on règle le mélange réactionnel à une valeur de pH de 5 à 9 ou de 6 à8;
c) on ajoute d'abord du charbon activé au mélange réactionnel traité de cette manière et on maintient le mélange réactionnel au reflux et on élimine ensuite le charbon activé du mélange réactionnel par filtration ;
d) on inocule ensuite au filtrat de la 2-fluoro-ara-adénine en l'absence d'une isolation intermédiaire de la 2-fluoro-ara-adénine brute, on refroidit une nouvelle fois la suspension résultante et on poursuit l'agitation ; et
e) enfin, on lave le gâteau solide isolé avec une solution aqueuse alcoolique et on sèche à une température supérieure sous vide en utilisant un gaz porteur.

2. Procédé selon la revendication 1, **caractérisé en ce que**, dans les étapes a) et e) du procédé, on utilise un mélange d'éthanol et d'eau à titre de solution alcoolique aqueuse.

3. Procédé selon l'une quelconque des revendications 1 et 2,
**caractérisé en ce que**, à l'étape a) du procédé, le travail est effectué dans une plage de températures de 35 à 45 °C ; ou
**caractérisé en ce que**, à l'étape a) du procédé, le travail est effectué dans une plage de températures de 37 à 43 °C.

4. Procédé selon l'une quelconque des revendications 1 à 3 ;
**caractérisé en ce que**, à l'étape d) du procédé, l'inoculation est mise en oeuvre dans une plage de températures de 40 à 75 °C ; ou
**caractérisé en ce que**, à l'étape d) du procédé, l'inoculation est mise en oeuvre dans une plage de températures de 50 à 60 °C ; ou
**caractérisé en ce que**, à l'étape d) du procédé, l'inoculation est mise en oeuvre dans une plage de températures de 52 à 58 °C.

5. Procédé selon l'une quelconque des revendications 1 à 4 ;
**caractérisé en ce que**, à l'étape d) du procédé, l'inoculation avec la 2-fluoro-ara-adénine est mise en oeuvre en une quantité de 0,05 à 1 kg ; ou
**caractérisé en ce que**, à l'étape d) du procédé, l'inoculation avec la 2-fluoro-ara-adénine est mise en oeuvre en une quantité de 0,05 à 0,2 kg.
